# EUROPEAN PATENT APPLICATION

(11) **EP 1 519 293 A2**
(43) Date of publication of application: **30.03.2005**
(21) Application number: 04017087.0
(22) Date of filing: 20.07.2004
(51) Int. Cl.: G06F 19/00

(54) **A medication administration system**

(30) Priority: 25.07.2003 US 490322 P; 19.12.2003 US 742232
(71) Applicant: Siemens Medical Solutions Health Services Corporation, Malvern, PA 19355 (US)
(72) Inventor: Miller, Raymond F., 19352 Lincol University, PA (US); Saeger, Deborah, 18103 Allentown, PA (US)
(74) Representative: French, Clive Harry

(57) **Abstract**

A system automatically updates a patient medication administration schedule in response to a healthcare worker entering data into an information system identifying a time at which a medication was administered to the patient. A medication administration information system comprises a processor for maintaining a schedule identifying a first nominal time for administering a first medication to a patient. The first medication is scheduled for administration to the patient subsequent to administration of a second medication to the patient. The processor updates the schedule by re-calculating the first nominal time for administering the first medication to the patient to provide a re-calculated time in response to received information identifying at least one of, (a) a late administration of the second medication to the patient and (b) an early administration of the second medication to the patient. The late and early administration being relative to a second scheduled nominal time for administering the second medication to the patient. A display generator initiates generation of data representing at least one display image including the re-calculated time for administering the first medication to the patient.

## Description

### Field of the Invention

This invention concerns an information system for scheduling administration of a medication to a patient.

### Background of the Invention

The administration of medication to a patient often does not run to schedule. The administration of continuous Intra-Venous (IV) infusions, for example, frequently run behind schedule, due to IV bag overfill, temporary stoppages to administer blood or other IV drugs, rate changes, occlusions, etc. It is difficult for an information system (such as a pharmacy system) to track the times at which administration of a medication such as an IV is initiated and the times future medications are due. Hospitals that attempt to collate medication administration tracking data typically either burden a nurse with extra reporting tasks, or require pharmacy personnel to conduct an inspection (e.g. of IVs being administered) in patient care in order to manually gather data. If a medication is not administered at a scheduled time but either later or earlier than the scheduled time, there is often a significant impact on resource management in a hospital such as within a pharmacy department. As an example, an IV may be prepared when it is not actually needed. Further, an IV medication may be extremely expensive and prepared with a patient-specific dose, or have a short time period of stability. Consequently, an IV medication may not be able to be reused and may be wasted if it is not used at the scheduled administration time. This inefficiency burdens a pharmacy department with a need to provide extra personnel to manufacture medications that are not used as well as the non-recoverable cost of these medications. Further, significant time and effort is expended in a pharmacy department in attempting to monitor changes or modifications to a patient medication. Personnel also need to be assigned to locate already prepared IV medications and to re-label, re-mix, or discard unusable IV medications and other medications. These tasks complicate the work of a pharmacy department and increase potential error. A system addresses the problems involved in scheduling and administering Intra-Venous (IV) and other medications to a patient and associated and derivative problems.

### Summary of the Invention

The present invention provides methods and apparatus as defined in the appended claims.

For example, a system automatically updates a patient medication administration schedule (e.g. maintained by a pharmacy) consistently with a medication order and prescribed frequency in response to a healthcare worker (e.g., a nurse) entering data into an information system identifying a time at which a medication was administered. A medication administration information system comprises a processor for maintaining a schedule identifying a first nominal time for administering a first medication to a patient. The first medication is scheduled for administration to the patient subsequent to administration of a second medication to the patient. The processor updates the schedule by re-calculating the first nominal time for administering the first medication to the patient to provide a re-calculated time in response to received information identifying at least one of, (a) a late administration of the second medication to the patient and (b) an early administration of the second medication to the patient. The late and early administration being relative to a second scheduled nominal time for administering the second medication to the patient. A display generator initiates generation of data representing at least one display image including the re-calculated time for administering the first medication to the patient.

### Brief Description of the Drawing

Figure 1 shows operational functions of a hospital, for example, including a medication administration information system, according to invention principles.
Figure 2 shows a user interface display image showing a medication administration schedule, according to invention principles.
Figure 3 shows a user interface display image showing a medication administration schedule including shifted medication administration times, according to invention principles.
Figure 4 shows a message communicated to a remote system (such as a pharmacy or medication order processing system) in response to update of a medication administration schedule with a shifted medication administration time, according to invention principles.
Figure 5 shows a Pharmacy application user interface display image identifying a particular patient medication with a shifted administration time, according to invention principles.
Figure 6 shows a Pharmacy application user interface display image detailing a shifted medication administration schedule for a medication identified in Figure 5, according to invention principles.
Figure 7 shows a user interface display image used for configuring a medication administration clinical safety check performed at time of administering a medication to a patient, according to invention principles.
Figure 8 shows a flowchart of a process used by a medication management information system in determining and processing shifted medication administration times in response to previous user entered medication administration times that deviate from schedule, according to invention principles.
Figure 9 shows a user interface display image indicating potential safety problems in administering a medication to a patient, according to invention principles.

### Detailed Description of Invention

Figure I shows departments and functions of a healthcare enterprise such as a hospital including a medication administration information system. Healthcare Information System (HIS) 12 incorporates medication administration system 13 (such as an executable application) and responds to user actions 10, by bidirectionally communicating with external systems 17-21 through interface engine 15. In response to a user such as a clinician entering a medication order 10 for a patient, medication administration system 13 in HIS 12 schedules delivery of the medication to a patient. The medication schedule for the patient is available for display to a user on PC 19 and data representing the medication schedule and other HIS and patient record data is stored in repository 14. Information is also entered by a user (such as a nurse or physician) recording actual time and date of administration of a medication to a patient via PC 19, for example. System 13 automatically updates a patient medication administration schedule maintained in HIS 12 (as part of a pharmacy management application, an order processing application or another application), by shifting scheduled medication administration times in response to a healthcare worker (e.g., a nurse) entering data via PC 19 identifying actual time and date of administration of a medication. This facilitates medication resource management and planning and reduces instances of medication overstocking or of under ordering. The updated schedule enables nurses to accurately identify medication (e.g., IV infusions) to be administered to patients and facilitates maintaining patients on their medication administration schedules.

System 13 advantageously improves patient safety by automatically adjusting patient medication (e.g., IV infusions) in order to maintain an accurate medication (e.g., infusion bottle) sequence. This reduces communications between nursing and pharmacy departments made to manually adjust and reprint labels and associated bar codes used in identifying a medication, in response to an early or late administration of a scheduled medication to a patient, for example. The system also improves Pharmacy operation by reducing waste resulting from expiration of medication (e.g., IV bottles/bags) because of patient medication administration getting out of sequence as well as the waste of resources involved in preparing, stocking and distributing expired medications. The system also increases patient safety by supporting on-line clinical medication safety checking in a Pharmacy (e.g., for allergies, drug interactions, conflicts, dosage and other errors), substantially at the time of medication administration.

A user enters data via PC 19 to determine maximum and minimum Shift thresholds that are used to denote both Early medication administration and Late medication administration in system 13. The administration of a medication at a time within a first time range prior to the original scheduled administration time for this medication (i.e., an Early administration), triggers system 13 to shift a scheduled time of administration of a subsequent medication. The first time range is bounded by a user defined minimum time duration value (the Early minimum Shift threshold value) and a user defined maximum time duration value (the Early maximum Shift threshold value) prior to the original scheduled administration time. The administration of a medication at a time within a second time range after the original scheduled administration time for this medication (i.e., a Late administration), also triggers system 13 to shift a scheduled time of administration of a subsequent medication. The second time range is bounded by a user defined minimum time duration value (the Late minimum Shift threshold value) and a user defined maximum time duration value (the Late maximum Shift threshold value) after the original scheduled administration time. A user is therefore able to largely independently set Early and Late Shift threshold minimum and maximum values (four separate values). These four Shift threshold values may be set for each medication (e.g., IV infusion). If the system 13 Shift threshold values are not set (and are null or zero valued, for example) for a particular medication, the medication administration schedule shift function is not turned on for that particular medication.

Messages conveying data determining a medication administration schedule for a patient are communicated by system 13 via interface engine 15 for use by pharmacy system 18, for example. Interface engine 15 may comprise a workflow processing application or other application supporting communication with external systems 17-21. A workflow as used herein comprises a sequence of tasks or operations that are scheduled for performance, or are being performed, by one or more entities including individuals, groups of individuals, or personnel assigned to perform particular functions or roles. External systems 17-21 comprise a laboratory system 17, pharmacy system 18 and financial application (such as for patient service tracking and billing) 21, for example, but may also encompass a broader range of systems including any system with which HIS 12 performs a transaction or data exchange. Further Healthcare Information System (HIS) 12 may comprise other types of information system such as a Clinical Information System or Critical Care Information System or another Information system. Further, in other embodiments HIS 12 may include non-healthcare information systems such as financial information systems provided that such a system involves performing a sequence of tasks that is susceptible to modification in response to a shift in a task schedule resulting from early or late performance of a prior task in the sequence.

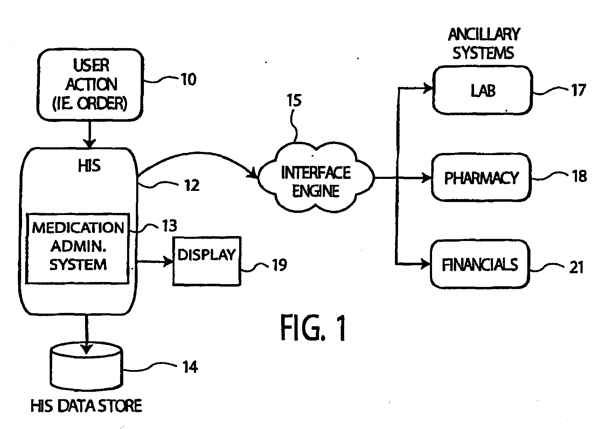

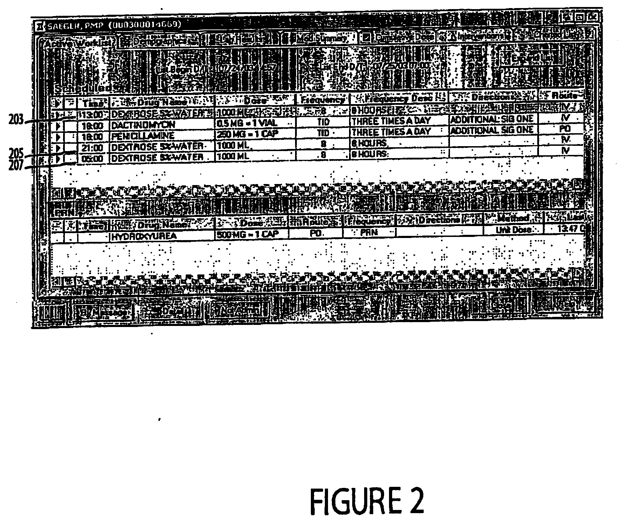

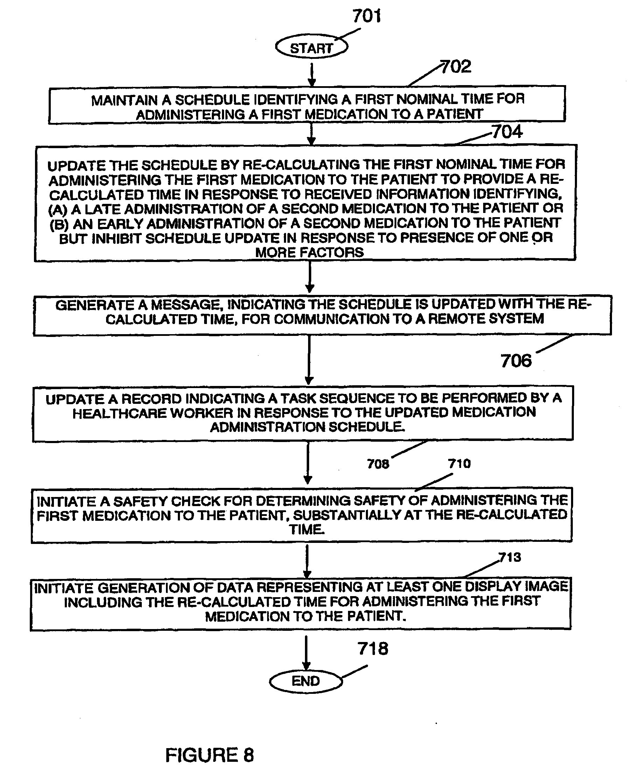

Figure 8 shows a flowchart of a process used by medication management information system 13 (Figure 1) in determining and processing shifted medication administration times in response to previous user entered medication administration times that deviate from schedule. In step 702 after the start at step 701, system 13 maintains a schedule identifying a nominal time for administering medication to a patient. The medication comprises, an IV infusion fluid, an orally administered medication, an injected medication or a medication applied externally to a patient's body, for example Figure 2 shows a user interface display image showing a medication administration schedule identifying that Dextrose 5%-Water is to be administered to a patient at nominal times 13:00, 21:00 and 5:00 (items 203, 205 and 207 respectively) in response to a physician order. In step 704, system 13 receives information for identifying a late or an early administration of a first medication and that is derived from a user entered time of medication administration or in another embodiment, derived from data comprising a bar code scan of a medication identifier and a bar code scan of a patient identifier.

In response to received information identifying a late or an early administration of a first medication to the patient relative to a firs nominal time, system 13 determines a value substantially representing a time difference between the first nominal time and a time of late administration or early administration of the first medication. System 13 determines whether the time difference value is within a predetermined time range. For this purpose system 13 determines if the time difference value exceeds a first predetermined threshold value (a corresponding Early or Late minimum Shift threshold value) and also whether the time difference value is less than a second predetermined threshold value (a corresponding Early or Late maximum Shift threshold value). If the time difference value is within the predetermined time range and schedule update is not inhibited, system 13 updates the schedule by re-calculating a second nominal time for administering a subsequent second medication to the patient to provide a re-calculated time in response to the received information identifying a late or an early administration of a first medication to the patient relative to first nominal time. The first and second medications are typically doses of the same medication administered at different times such as Dextrose 5%-Water of items 203, 205 and 207 of Figure 2. However, the first and second medications may also be different.

A user configures system 13 with Early and Late minimum and maximum Shift threshold values for a particular scheduled medication administration to a particular patient via a display menu illustrated as follows.
SELECT FILE ==>PMD 'PHARMACY METHOD DEFAULT FILE'
COMMAND ==>REVISE
METHOD ==>LVP
COMPONENT# ==>ALL

- 1: DESCRIPTION ==>LARGE VOLUME PARENTERAL =>
- 2: METHOD CHARGE ==>5.00 ==>
- 3: STOP DAYS ==>30 ==>
- 4: SET TYPE ==>
- 6: ROUTE ==>IV ==>
- 7: DUR TRACKING? ==>YES ==>
- 9: METHOD ABBR. ==>LVP ==>
- 10: METHOD CDM ==>
- 11: INSURANCE CODE ==>00 ==>
- 12: SKILL LEVEL ==>
- 13: EARLY SHIFT THRESHOLD==> 180/360 ==>
- 14: LATE SHIFT THRESHOLD==>120/360 ==>

A user employs the previous menu to select Late minimum and maximum Shift threshold values 120/360 comprising threshold values of 2 and 6 hours late respectively, for example. System 13 limits a maximum Shift threshold value to be less than or equal to 1440 minutes or another value in a different embodiment.

System 13 inhibits update of the medication administration schedule in response to a determination of at least one of, (a) an order for administration of the medication has been discontinued, (b) an order for administration of the medication has reached its stop date, (c) an order for administration of the medication involves repeated separate administration operations, (d) an order for administration of the medication has been revised, (e) an order for administration of the medication indicates no further administration is required and (f) an order for administration of the medication initiates administration after the recalculated time.

System 13 re-calculates a nominal time for administering medication to the patient by shifting scheduled medication administration times by a period substantially comprising a time duration of the late administration of the medication to the patient or a time duration of the early administration of a medication to the patient. System 13 also approximates shifted times to predetermined interval boundary times such as 1, 5, 10, 15, 30 or 60 minute intervals, for example. In another embodiment, system 13 uses shifted scheduled medication administration times that are not approximated to boundary times.

In step 706, system 13 generates a message for communication to update a remote medication order processing system and a medication management information system such as a pharmacy management executable application, for example. The message indicates the medication administration schedule is updated with the re-calculated nominal time for administering the medication. The message is usable by the medication management information system to indicate a change in time for preparation of a medication and to adjust time for distribution of a prepared medication and also to initiate preparation of a label for use on a prepared medication.

The prepared label comprises a medication administration shift notice label identifying that a medication has a shifted administration schedule and including a date and time of an occurrence that initiated the automatic shift, an administration frequency, name of a person who recorded the occurrence that initiated the automatic shift, an original date and time of the first occurrence that was shifted automatically and a time shift amount. Other information on the label includes, a healthcare facility identifier, patient identifier, patient room and bed identifiers, patient nurse station and medication order number.

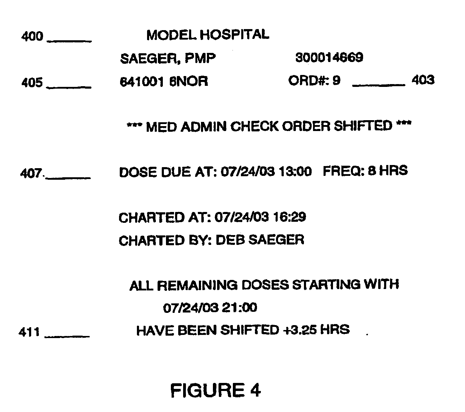

In addition, an inventory manager in the medication management information system updates a medication inventory in response to the message. Figure 4 shows a message communicated to a remote system (such as a pharmacy or medication order processing system) indicating a shifted medication administration time. The message identifies the healthcare provider organization 400, patient, medication administration order 403, particular medication and time due as well as frequency of administration (items 405 and 407) and the time period 411 by which the particular medication administration time (and subsequent doses) have been shifted (3 hours 15 minutes (3.25 hours) in this example).

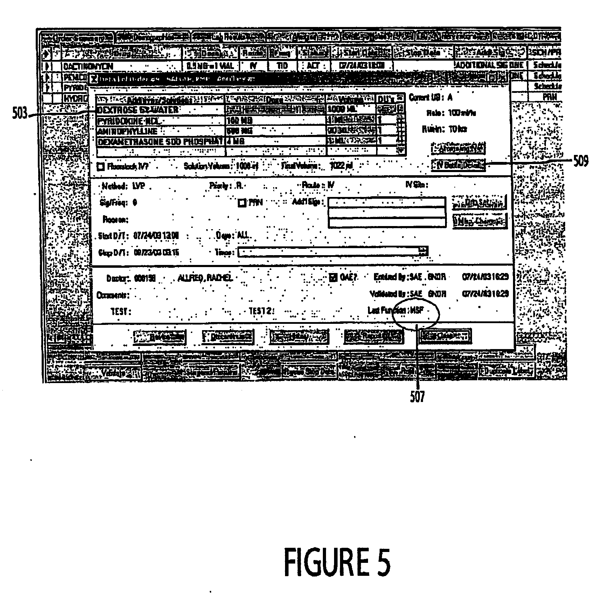

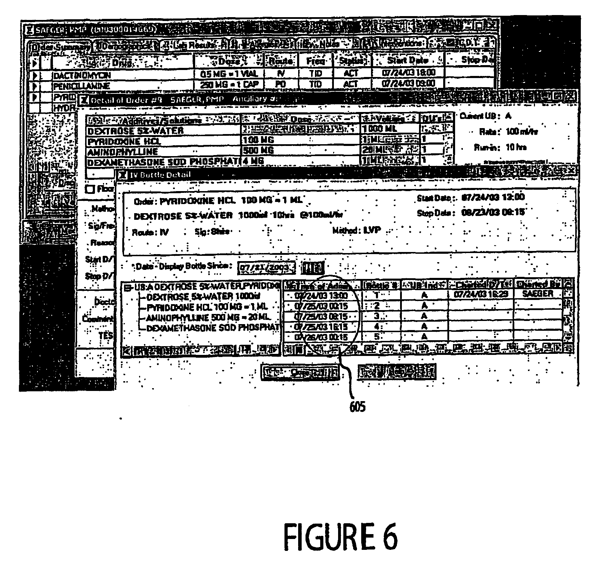

Figure 5 shows a Pharmacy application user interface display image identifying composition of a particular patient medication 503 with a shifted administration time. The particular medication 503, in this example, is a composite IV infusion comprising Dextrose 5%-Water, Pyridoxene HCL, Aminophylline and Dexamethasone. The Pharmacy application display image identifies a last function performed for the order for administration of this IV infusion 503. Specifically, the display image indicates that the last function performed (item 507) for this order was a medication administration schedule shift (MSF indicating Medication Administration Check Shift). In addition, system 13 initiates generation of a report detailing Medication Administration Check Shift for a defined timeframe (such as the past 24 hours, for example) in response to user command. Upon user selection of IV Bottle Detail button 509, the medication administration schedule for this IV is shown in Figure 6, for example. Specifically, item 605 identifies the shifted medication administration schedule for the IV infusion identified in Figure 5.

System 13 in step 708 updates a record indicating a task sequence to be performed by a healthcare worker (or by automatic processing) in response to the updated medication administration schedule. The times of medication administration need to be synchronized between a pharmacy and nursing departments to coordinate drug dispensing and administration. A nurse may frequently desire to make minor changes to times of administration of a medication to accommodate a patient's daily care routine including meal times, therapies that require the patient to be away from the patient care area and personal patient preferences and other items. For example, a drug may be ordered twice a day with scheduled administration times of 10:00 AM and 6:00 PM. However, a nurse is aware that this patient is scheduled for physical therapy from 10-11 AM each day, and therefore the nurse changes the morning time of administration to 9:30 AM. A similar situation may exist for orders having a standard frequency, an unusual frequency, or an unspecified schedule.

System 13 allows a nurse to make a minor revision within the guidelines of an existing ordered medication administration frequency and eliminates the need to ask a pharmacist or physician to make a change in a pharmacy system or Order Entry system. System 13 further facilitates coordinated medication dispensing and distribution between pharmacy and nursing departments based on an updated schedule containing shifted administration times. System 13 facilitates a nurse documenting a change in a clinical system and reduces instances in which a healthcare worker may ignore medication administration schedule data to give a medication at the worker's discretion. Even a relatively small shift in medication administration time (e.g., 30 minutes) may have significant ramifications to dispensing, distribution, inventory management, people and other resource management and associated workflow task sequences. For example, a dose due at 3:00PM is dispensed by pharmacy and placed on a unit-dose cart delivered at 3:00 PM. However, nursing desires to give this dose at 2:30 PM, and it is not available in the patient drawer of the unit-dose cart currently on the nursing unit. This causes significant disruption to healthcare delivery operations.

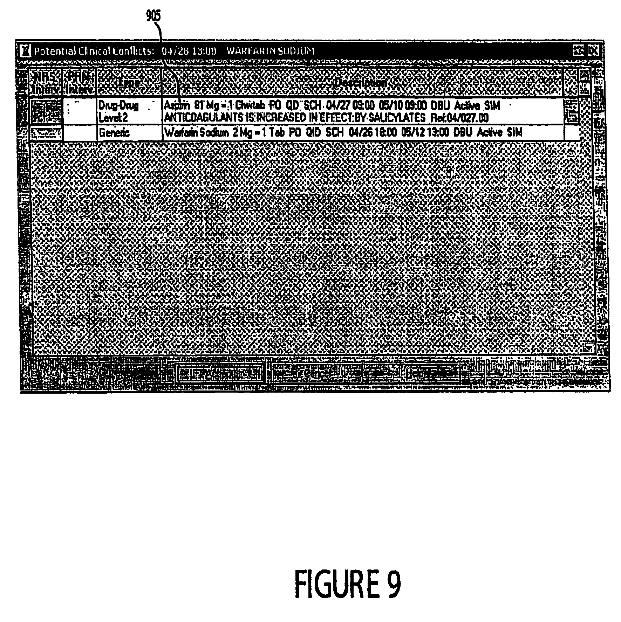

In step 710, system 13 initiates a clinical safety check for determining safety of administering the version of medication to the patient that is current substantially at the re-calculated time. The clinical safety check includes, determining whether the patient is allergic to the medication, determining whether the medication interacts with another medication taken by the patient and determining whether the medication conflicts with another medication taken by the patient. The clinical safety check also includes checking for, generic duplicate medications previously prescribed, other duplicate medications previously prescribed, Pediatric Precautions, Geriatric Precautions, Laboratory Rules and Warnings, Rules Engine Warnings, Minimum and Maximum dose Checking and Cumulative Total Dose checking. System 13 initiates generation of a user interface display image, exemplified in Figure 9, indicating potential safety problems 905 associated with administering a medication to a patient.

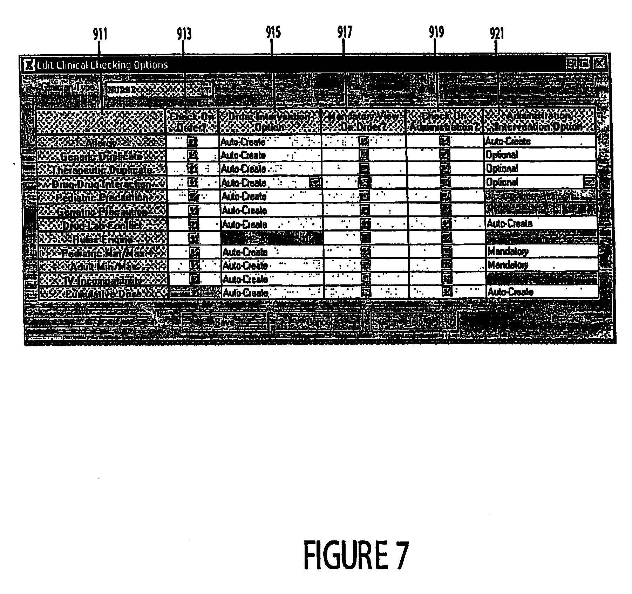

Figure 7 shows a user interface display image used for configuring a medication administration clinical safety check performed at time of administering a medication to a patient. The Figure 7 user interface image enables a Clinician of a particular Type to determine whether a particular clinical safety check is performed and whether a Clinical Intervention is required as a result of a conflict being identified by performing the particular clinical safety check. The Figure 7 user interface image enables a Clinician to determine whether a Clinical Intervention is mandatory, optional, or automatically initiated and whether a user needs to view identified conflict results. The Figure 7 user interface image also enables separate clinical safety checks to be configured to be performed both upon user order entry and upon medication administration. Specifically, the image enables clinical safety checks to be configured to be performed upon user entry of an order for administration of a particular medication to a patient and substantially upon administration of a medication.

Column 911 lists various safety checks that may be selected and configured. Columns 915 and 921 are used to associate manual intervention actions with individual safety checks. Column 915 enables a user to initiate an intervention action associated with an order (and with an order processing application) for administration of a medication to a patient. Column 921 enables a user to initiate an intervention action associated with system 13 and administration of a medication to a patient. In other embodiments system 13 may include an order processing executable application or the medication administration and order processing applications may be separate. Column 913 enables a user to select individual safety checks to be performed in response to user entry of an order for administration of a medication to a patient in an order processing application. Column 917 enables a user to select that it is mandatory to view results of a performed clinical safety check prior to the initiation of an order for a particular medication for a patient. Column 919 enables a user to select that a clinical safety check is performed, prior to and substantially near, or at, the time of administration of a particular medication to a patient.

System 13 maintains a database identifying clinical safety checks and results performed as well as user entered commands overriding clinical safety check results. The database further supports determination of an audit trail for use in identifying users entering commands associated with clinical safety checks that are performed, safety check results overridden, medication administration times that have been shifted and other medication administration times. System 13 initiates generation of reports identifying clinical safety checks that are performed, safety check results overridden, medication administration times that have been shifted, other medication administration times and associated audit trail data, in response to user command. In step 713, system 13 initiates generation of data representing at least one display image including the re-calculated time for administering the medication to the patient.

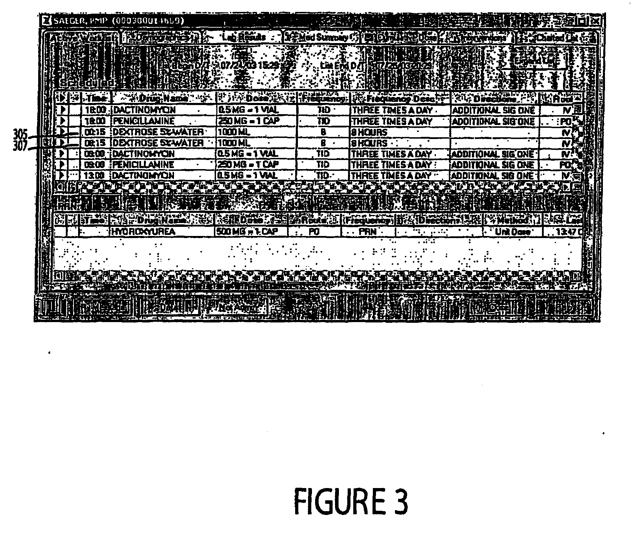

Figure 3 shows a user interface display image showing a medication administration schedule including the Dextrose 5%-Water medication administration 21:00 and 05:00 times (of the Figure 2 schedule) shifted to corresponding 00:15 and 08:15 times (items 305 and 307 of Figure 3). This shift is performed by system 13 in response to a late administration by 3 hours 20 minutes of the medication scheduled for 13:00 in the Figure 2 schedule. A nurse administers the Dextrose 5%-Water medication scheduled for13:00 (in the schedule of Figure 2) at 16:20pm (3 hours and 20 minutes late). System 13 determines the 3 hours and 20 minutes time difference is within a predetermined late time range bounded by a Late minimum Shift threshold and a Late maximum Shift threshold (2 and 6 hours late respectively in this example) and that a schedule update is not inhibited. Consequently, system 13 updates the schedule by re-calculating the nominal time for administering the medication to the patient by a time period used to shift future scheduled medication administration times. System 13 provide a re-calculated time in response to the received information identifying a late or an early administration of a medication to the patient relative to the nominal time.

System 13 subtracts a scheduled medication administration date and time from an actual date and time of administration of the medication (e.g., as presented on a medical chart), to determine in hours and minutes how late or early it is administered. In the example illustrated in the schedules of Figures 2 and 3, the Dextrose 5%-Water medication scheduled in Figure 2 for administration at 13:00 is actually administered at 16:20 (3hrs and 20 minutes late). System 13 approximates the 3 hours 20 minutes time difference to the closest 15 minute boundary (3 hours 15 minutes in this example), and this is the amount it shifts subsequent scheduled Dextrose 5%-Water medication IV administration times. The associated order displayed by system 13 in Figure 3 shows the subsequent Dextrose 5%-Water medication administration times shifted by 3 hours 15 minutes to 00:15 and 08:15 (items 305 and 307 of Figure 3 corresponding to scheduled 21:00 and 05:00 medication administration times of the Figure 2 schedule). The process of Figure 8 terminates at step 718.

The user interface display images, systems and processes presented in Figures 1-9 are not exclusive. Other user interface and processing systems may be derived in accordance with the principles of the invention to accomplish the same objectives. Although this invention has been described with reference to particular embodiments, it is to be understood that the embodiments and variations shown and described herein are for illustration purposes only. Modifications to the current design may be implemented by those skilled in the art, without departing from the scope of the invention. An event scheduling and schedule shift management system and associated functions according to invention principles may be used in other applications to support improved management of resources associated with a managed schedule.

## Claims

1. A medication administration information system, comprising:
a processor for,
maintaining a schedule identifying a first nominal time for administering a first medication to a patient, said first medication being scheduled for administration to said patient subsequent to administration of a second medication to said patient and
updating said schedule by re-calculating said first nominal time for administering said first medication to said patient to provide a re-calculated time in response to received information identifying at least one of, (a) a late administration of said second medication to said patient and (b) an early administration of said second medication to said patient, said late and early administration being relative to a second scheduled nominal time for administering said second medication to said patient; and
a display generator for initiating generation of data representing at least one display image including said re-calculated time for administering said first medication to said patient.

2. A system according to claim 1, wherein
said processor re-calculates said first nominal time for administering medication to said patient by shifting a scheduled administration time of said first medication by at least one of, (a) a period substantially comprising a time duration of said late administration of said second medication to said patient and (b) a period substantially comprising a time duration of said early administration of said second medication to said patient.

3. A system according to claim 1, wherein
said processor re-calculates said first nominal time for administering said first medication to said patient by shifting a scheduled medication administration time and approximating a shifted time to a predetermined interval boundary time and
said predetermined interval boundary time is a time occurring at an interval boundary comprising at least one of, (a) a 5 minute interval boundary, (b) a 10 minute interval boundary, (c) a 15 minute interval boundary, (d) a 30 minute interval boundary, (e) a one minute interval boundary and (f) a 1 hour interval boundary.

4. A system according to any preceding claim, wherein
said processor determines a value substantially representing a time difference between said second nominal time for administering said second medication and at least one of, (a) a time of said late administration of said second medication and (b) a time of said early administration of said second medication and
said processor re-calculates said first nominal time if said value exceeds a first predetermined threshold value and
said processor re-calculates said first nominal time if said value is less than a second predetermined threshold value.

5. A system according to any preceding claim, including
a message generator for generating a message for communication to a remote system, said message indicating said schedule is updated with said re-calculated time and
said message is usable by a medication management information system to indicate a change in time for preparation of a medication.

6. A system according to claim 5, wherein
said message is communicated to update a medication order processing system to indicate said re-calculated time for administering said first medication and is usable by a medication management information system to adjust time for distribution of a prepared medication.

7. A system according to claim 5 or claim 6, including
said message is used in preparing a label for use on a prepared medication.

8. A system according to any preceding claim, wherein
said processor inhibits update of said schedule in response to a determination of at least one of, (a) an order for administration of said first medication has been discontinued, (b) an order for administration of said first medication has reached its stop date, (c) an order for administration of said first medication requires repeated separate administration operations, (d) an order for administration of said first medication has been revised, (e) an order for administration of said first medication indicates no further administration is required and (f) an order for administration of said first medication initiates administration after said recalculated time.

9. A system according to any preceding claim, including
an inventory manager for updating a medication inventory in response to said updated medication administration schedule and wherein
said medication comprises at least one of, (a) an IV infusion fluid, (b) an orally administered medication, (c) an injected medication and (d) a medication applied externally to a patient's body.

10. A system according to any preceding claim, wherein
said first medication and said second medication are separately scheduled doses of the same or different medications.

11. A medication administration information system, comprising:
a processor for,
maintaining a schedule identifying a first nominal time for administering a first medication to a patient, said first medication being scheduled for administration to said patient subsequent to administration of a second medication to said patient and
updating said schedule by re-calculating said first nominal time for administering said first medication to said patient to provide a re-calculated time in response to received information identifying at least one of, (a) a late administration of said second medication to said patient and (b) an early administration of said second medication to said patient, said late and early administration being relative to a second scheduled nominal time for administering said second medication to said patient; and
a task manager for updating a record indicating a task sequence to be performed by a healthcare worker in response to said updated schedule.

12. A system according to claim 11, wherein
said processor initiates a clinical safety check, substantially at said re-calculated time, said clinical safety check being for determining safety of administering said first medication to said patient and
said clinical safety check comprises at least one of, (a) a determination whether said patient is allergic to said first medication, (b) a determination whether said first medication interacts with another medication taken by said patient and (c) a determination whether said first medication conflicts with another medication taken by said patient.

13. A system according to claim 11 or claim 12, wherein
said received information is derived from data comprising a bar code scan of a second medication identifier and a bar code scan of a patient identifier and
said received information is derived from a user entered time of second medication administration.

14. A method for monitoring medication administration, comprising the activities of:
maintaining a schedule identifying a first nominal time for administering a first medication to a patient, said first medication being scheduled for administration to said patient subsequent to administration of a second medication to said patient;
updating said schedule by re-calculating said first nominal time for administering said first medication to said patient to provide a re-calculated time in response to received information identifying at least one of, (a) a late administration of said second medication to said patient and (b) an early administration of said second medication to said patient, said late and early administration being relative to a second scheduled nominal time for administering said second medication to said patient; and
initiating generation of data representing at least one display image including said re-calculated time for administering said first medication to said patient.

15. A method for managing medication administration, comprising the activities of:
maintaining a schedule identifying a first nominal time for administering a first medication to a patient, said first medication being scheduled for administration to said patient subsequent to administration of a second medication to said patient;
updating said schedule by re-calculating said first nominal time for administering said first medication to said patient to provide a re-calculated time in response to received information identifying at least one of, (a) a late administration of said second medication to said patient and (b) an early administration of said second medication to said patient, said late and early administration being relative to a second scheduled nominal time for administering said second medication to said patient; and
updating a record indicating a task sequence to be performed by a healthcare worker in response to said updated schedule.
